# NOUVEAU FASCICULE DE BREVET EUROPEEN

(11) **EP 1 047 380 B2**
(45) Date de publication et mention de la décision concernant l'opposition: **18.02.2009**
(45) Mention de la délivrance du brevet: 06.05.2004
(21) Numéro de dépôt: 98963595.8
(22) Date de dépôt: 22.12.1998
(51) Int. Cl.: A61K 8/66

(54) **COMPOSITION DE TEINTURE D'OXYDATION DES FIBRES KERATINIQUES CONTENANT UNE LACCASE ET PROCEDE DE TEINTURE METTANT EN OEUVRE CETTE COMPOSITION**
OXIDATIONSFÄRBEMITTEL FÜR KERATINFASERN, DAS EINE LACCASE ENTHÄLT, UND VERFAHREN ZUR FÄRBUNG MIT DIESEM MITTEL
KERATINOUS FIBRE OXIDATION DYEING COMPOSITION CONTAINING A LACCASE AND DYEING METHOD USING SAME

(30) Priorité: 13.01.1998 FR 9800260
(43) Date de publication de la demande: 02.11.2000
(73) Titulaire: L'ORÉAL, 75008 Paris (FR)
(72) Inventeur: LANG, Gérard, F-95390 Saint Prix (FR); COTTERET, Jean, F-78480 Verneuil sur Seine (FR)
(74) Mandataire: Miszputen, Laurent
(86) Numéro de dépôt international: PCT/FR1998/002830
(87) Numéro de publication internationale: WO 1999/036038

(56) Documents cités:
- EP-A- 0 241 716
- EP-A- 0 504 005
- EP-A- 0 634 162
- EP-A- 0 634 164
- EP-A- 0 667 143
- EP-A- 0 791 352
- EP-A- 0 795 313
- WO-A1-98/22078
- DE-A- 2 155 359
- DE-A- 4 440 955
- FR-A- 2 694 018

## Description

L'invention a pour objet une composition pour la teinture d'oxydation des fibres kératiniques, et en particulier des fibres kératiniques humaines telles que les cheveux comprenant, dans un milieu approprié pour la teinture, du 3-méthyl 4-amino phénol à titre de base d'oxydation, au moins un coupleur, et au moins une enzyme de type laccase, ainsi que le procédé de teinture mettant en oeuvre cette composition.

Il est connu de teindre les fibres kératiniques et en particulier les cheveux humains avec des compositions tinctoriales contenant des précurseurs pour la coloration d'oxydation, en particulier des ortho ou paraphénylènediamines, des ortho ou paraaminophénols, des bases hétérocycliques, appelés généralement bases d'oxydation. Les précurseurs pour la coloration d'oxydation, (bases d'oxydation), sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé dans le brevet US 3 251 742, les demandes de brevet FR-A-2 112 549, FR-A-2 694 018, EP-A-0 504 005, WO95/07988, WO95/33836, WO95/33837, WO96/00290, WO97/19998 et WO97/19999 de teindre les fibres kératiniques avec des compositions comprenant au moins colorant d'oxydation, ou au moins un précurseur de mélanine, en association avec des enzymes du type lactase; lesdites compositions étant mises en contact avec l'oxygène de l'air. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes à la fois sur le plan de l'homogénéité de la couleur répartie le long de la fibre (unisson), sur le plan de la chromaticité (luminosité) et de la puissance tinctoriale.

Or, la demanderesse vient maintenant de découvrir qu'il est possible d'obtenir de nouvelles teintures, capables de conduire à des colorations puissantes sans engendrer de dégradation significative des fibres kératiniques, peu sélectives et résistant bien aux diverses agressions que peuvent subir les fibres, en associant du 3-méthyl 4-amino phénol à titre de base d'oxydation, au moins une enzyme de type laccase, et au moins un coupleur.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- du 3-méthyl 4-amino phénol et/ou au moins l'un de ses sels d'addition avec un acide, à titre de base d'oxydation,
- au moins une enzyme de type laccase; et
- au moins un coupleur.

La composition tinctoriale prête à l'emploi conforme à l'invention conduit à des colorations puissantes présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

Le 3-méthyl 4-amino phénol et/ou son ou ses sels d'addition avec un acide représentent de préférence de 0,0005 à 12 % en poids environ du poids total de la composition tinctoriale conforme à l'invention, et encore plus préférentiellement de 0.005 à 6 % en poids environ de ce poids.

La ou les laccases utilisées dans la composition tinctoriale prête à l'emploi conforme à l'invention peuvent notamment être choisies parmi les laccases d'origine végétale, d'origine animale, d'origine fongique (levures, moisissures, champignons) ou d'origine bactérienne, les organismes d'origine pouvant être mono- ou pluricellulaires. La ou les laccases utilisées dans la composition tinctoriale prête à l'emploi conforme à l'invention peuvent également être obtenues par biotechnologie.

Parmi les laccases d'origine végétale utilisables selon l'invention, on peut citer les laccases produites par des végétaux effectuant la synthèse chlorophyllienne telles que celles indiquées dans la demande de brevet FR-A-2 694 018.

On peut notamment citer les laccases présentes dans les extraits d'Anacardiacées tels que par exemple les extraits de Magnifera indica, de Schinus molle ou de Pleiogynium timoriense ; dans les extraits de Podocarpacées ; de Rosmarinus off. ; de Solanum tuberosum ; d'iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

Parmi les laccases d'origine fongique, éventuellement obtenues par biotechnologie, utilisables selon l'invention, on peut citer la ou les laccases issues de Polyporus versicolor, de Rhizoctonia praticola et de Rhus vernicifera telles que décrites par exemples dans les demandes de brevet FR-A-2 112 549 et EP-A-504005 ; les laccases décrites dans les demandes de brevet WO95/07988, WO95/33836, WO95/33837, WO96/00290, WO97/19998 et WO97/19999, dont le contenu fait partie intégrante de la présente description comme par exemple la ou les laccases issues de Scytatidium, de Polyporus pinsitus, de Myceliophtora thermophila, de Rhizoctonia solani, de Pyricularia orizae, et leurs variantes. On peut également citer la ou les laccases issues de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, dé Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

On choisira plus préférentiellement les laccases d'origine fongiques, éventuellement obtenues par biotechnologie.

L'activité enzymatique des laccases utilisées conformément à l'invention et ayant la syringaldazine parmi leurs substrat peut être définie à partir de l'oxydation de la syringaldazine en condition aérobie. L'unité Lacu correspond à la quantité d'enzyme catalysant la conversion de 1 mmole de syringaldazine par minute à un pH de 5,5 et à une température de 30°C. L'unité U correspond à la quantité d'enzyme produisant un delta d'absorbance de 0,001 par minute, à une longueur d'onde de 530 nm, en utilisant la syringaldazine comme substrat, à 30°C et à un pH de 6,5. L'activité enzymatique des laccases de l'invention peut aussi être définie à partir de l'oxydation de la paraphénylénediamine. L'unité ulac correspond à la quantité d'enzyme produisant un delta d'absorbance de 0,001 par minute, à une longueur d'onde de 496,5 nm, en utilisant la paraphénylènediamine comme substrat (64 mM), à 30°C et à un pH de 5.

Selon l'invention, on préfère déterminer l'activité enzymatique en unités ulac.

La composition tinctoriale conforme à l'invention renferme un ou plusieurs coupleurs de façon à modifier ou à enrichir en reflets les nuances obtenues en mettant en oeuvre le 3-méthyl 4-amino phénol.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-N-(β-hydroxyéthyl)amino 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide.

Le ou les coupleurs représentent de préférence de 0,0001 à 8 % en poids environ du poids total de la composition tinctoriale et encore plus préférentiellement de 0,005 à 5 % en poids environ de ce poids.

La composition tinctoriale conforme à l'invention peut également renfermer, en plus du 3-méthyl 4-amino phénol et/ou de ses sels d'addition avec un acide et du ou des coupleurs, au moins une base d'oxydation additionnelle pouvant être choisie parmi les bases d'oxydation utilisées de façon classique pour la teinture d'oxydation. Elles peuvent notamment être choisies parmi les paraphénylènediamines, les bases doubles, les para-aminophénols, les ortho aminophénols et les bases d'oxydation hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphènylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylénediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphènylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphényiènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, la ou les bases d'oxydation additionnelles représentent de préférence de 0,0005 à 12 % en poids environ du poids total de la composition tinctoriale conforme à l'invention, et encore plus préférentiellement de 0,005 à 6 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

La composition tinctoriale conforme à l'invention peut en outre contenir un ou plusieurs colorants directs.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau.

Le pH de la composition prête à l'emploi conforme à l'invention est choisi de telle manière que l'activité enzymatique de la laccase soit suffisante. Il est généralement compris entre 4 et 11 environ, et de préférence entre 6 et 9 environ. Il peut être ajusté à la valeur désirée au moyen d'agents acidifiants ou alcalinisants habituellement utilisés en teinture des fibres kératiniques.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères, des agents antioxydants, des enzymes différentes des laccases utilisées conformément à l'invention telles que par exemples des peroxydases ou des oxydo-réductases à 2 électrons, des agents de pénétration, des agents séquestrants, des parfums, des tampons, des agents dispersants, des agents épaississants, des agents filmogènes, des agents conservateurs, des agents opacifiants, des vitamines.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le 3-méthyl 4-amino phénol ou les coupleurs et éventuellement les colorants d'oxydation additionnels et la ou les enzymes de type laccase sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, du 3-méthyl 4-amino phénol et/ou au moins l'un de ses sels d'addition avec un acide, et d'autre part, une composition (B) renfermant, dans un milieu approprié pour la teinture, au moins une enzyme de type laccase, puis à procéder à leur mélange au moment de l'emploi, ledit mélange comprenant au moins un coupleur, avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, ledit mélange comprenant au moins un coupleur, tel que les dispositifs décrits dans le brevet FR-2 586 913 au nom de la demanderesse.

Les exemples qui suivent sont destinés à illustrer l'invention sans pour autant en limiter la portée.

### EXEMPLE DE TEINTURE

On a préparé la composition tinctoriale suivante :
- 3-méthyl 4-amino phénol 0,25 g
- 5-N-(β-hydroxyéthyl)amino 2-méthyl phénol 0,30 g
- Laccase issue de Rhus vernicifera à 180 unités / mg vendue par la société ICN 1,8 g
- Alkyl (C₈-C₁₀) polyglucoside en solution aqueuse à 60 % de matière active (M.A.), vendu sous la dénomination ORAMIX CG110 ® par la société SEPPIC 8,0 g
- Ethanol 20 g
- Agent de pH q.s. pH 6,5
- Eau déminéralisée q.s.p. 100 g

La composition tinctoriale prêtes à l'emploi décrite ci-dessus a été appliquée sur des mèches de cheveux gris naturels à 90 % de blancs pendant 40 minutes, à une température de 30°C. Les cheveux ont ensuite été rincés, puis séchés.

Les cheveux ont été teints dans une nuance doré cuivré.

Dans la composition tinctoriale décrite ci-dessus, la laccase de Rhus vernicifera à 180 unités / mg, vendue par la société Sigma peut être remplacée par 1,0 g de laccase de Pyricularia orizae à 100 unités / mg vendue par la société ICN.

## Revendications

1. Composition prête à l'emploi, pour la teinture d'oxydation des fibres kératiniques et en particulier des fibres kératiniques humaines telles que les cheveux, **caractérisée par le fait qu'**elle comprend, dans un milieu approprié pour la teinture :
- du 3-méthyl 4-amino phénol et/ou au moins l'un des ses sels d'addition avec un acide, à titre de base d'oxydation,
- au moins une enzyme de type laccase; et
- au moins un coupleur.

2. Composition selon la revendication 1, **caractérisée par le fait que** le 3-méthyl 4-amino phénol et/ou son ou ses sels d'addition avec un acide représentent de 0,0005 à 12 % en poids du poids total de la composition tinctoriale.

3. Composition selon la revendication 2, **caractérisée par le fait que** le 3-méthyl 4-amino phénol et/ou son ou ses sels d'addition avec un acide représentent de 0,005 à 6 % en poids du poids total de la composition tinctoriale.

4. Composition selon rune quelconque des revendications 1 à 3, **caractérisée par le fait que** la laccase est choisie parmi les laccases d'origine végétale, d'origine animale, d'origine fongique ou d'origine bactérienne et parmi les laccases obtenues par biotechnologie.

5. Composition selon la revendication 4, **caractérisée par le fait que** la laccase est d'origine végétale et choisie parmi les laccases présentes dans les extraits d'Anacardiacées : de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotrope hypopithys (sucepin), d'Aesculus sp. ; d'Acer pseudoplatanus ; de Prunus persica et de Pistacia palaestina.

6. Composition selon la revendication 4, **caractérisée par le fait que** la laccase est d'origine microbienne ou obtenue par biotechnologie.

7. Composition selon la revendication 6, **caractérisée par le fait que** la laccase est choisie parmi les laccases issues de Polyporus versicolor, de Rhizoctonia praticola, de Rhus vernicifera, de Scytalidium, de Polyporus pinsitus, de Myceliophtora thermophila, de Rhizoctonia solani, de Pyricularia orizae, de Trametes versicolor, de Fomes fomentarlus, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

8. Composition selon l'une quelconque des revendications précédentes, **caractérisée par le fait que** la quantité de laccase(s) est comprise entre 0,5 et 200 Lacu pour 100 g de composition tinctoriale.

9. Composition selon l'une quelconque des revendications précédentes, **caractérisée par le fait que** le ou les coupleurs sont choisis parmi les métaphénylènediamines, les méta-aminophénols, les métadiphénols et les coupleurs hétérocycliques.

10. Composition selon la revendication 9, **caractérisée par le fait que** les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-N-(β-hydroxyéthyl)amino 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide.

11. Composition selon l'une quelconque des revendications précédentes, **caractérisée par le fait que** le ou les coupleurs représentent de 0,0001 à 8 % en poids du poids total de la composition tinctoriale.

12. Composition selon la revendication 11, **caractérisée par le fait que** le ou les coupleurs représentent de 0,005 à 5 % en poids du poids total de la composition tinctoriale.

13. Composition selon l'une quelconque des revendications précédentes, **caractérisée par le fait qu'**elle renferme au moins une base d'oxydation additionnelle choisie parmi les paraphénylènediamines, les bases doubles, les para-aminophénols, les ortho aminophénols et les bases d'oxydation hétérocycliques.

14. Composition selon la revendication 13, **caractérisée par le fait que** la ou les bases d'oxydation additionnelles représentent de 0,0005 à 12 % en poids du poids total de la composition tinctoriale.

15. Composition selon la revendication 14, **caractérisée par le fait que** la ou les bases d'oxydation additionnelles représentent de 0,005 à 6 % en poids du poids total de la composition tinctoriale.

16. Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

17. Composition selon l'une quelconque des revendications précédentes, **caractérisée par le fait que** le milieu approprié pour la teinture est constitué par de l'eau ou par un mélange d'eau et d'au moins un solvant organique.

18. Composition selon l'une quelconque des revendications précédentes, **caractérisée par le fait qu'**elle présente un pH compris entre 4 et 11.

19. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans rune quelconque des revendications précédentes, pendant un temps suffisant pour développer la coloration désirée.

20. Procédé selon la revendication 19, **caractérisé par le fait qu'**il comporte une étape préliminaire consistant à stocker sous forme séparée, d'une part, une composition (A) comprenant, dans un milieu approprié pour la teinture du 3-méthyl 4-amino phénol et/ou au moins un de ses sels d'addition avec un acide à titre de base d'oxydation et, d'autre part, une composition (B) renfermant, dans un milieu approprié pour la teinture, au moins une enzyme de laccase, puis à procéder à leur mélange au moment de l'emploi avant d'appliquer ce mélange sur les fibres kératiniques.

21. Dispositif à plusieurs compartiments ou "kit" de teinture, **caractérisé par le fait qu'**il comporte un premier compartiment renfermant la composition (A) telle que définie dans la revendication 20 et un second compartiment renfermant la composition (B) telle que définie dans la revendication 20.

## Claims

1. Ready-to-use composition for the oxidation dyeing of keratin fibres, and in particular of human keratin fibres such as the hair, **characterized in that** it comprises, in a medium which is suitable for dyeing:
- 3-methyl-4-aminophenol and/or at least one of the addition salts thereof with an acid, as oxidation base,
- at least one laccase-type enzyme; and
- at least one coupler.

2. Composition according to. Claim 1, **characterized in that** the 3-methyl-4-aminophenol and/or the addition salt(s) thereof with an acid represent from 0.0005% to 12% by weight relative to the total weight of the dye composition.

3. Composition according to Claim 2, **characterized in that** the 3-methyl-4-aminophenol and/or the addition salt(s) thereof with an acid represent from 0.005% to 6% by weight relative to the total weight of the dye composition.

4. Composition according to any one of Claims 1 to 3, **characterized in that** the laccase is chosen from laccases of plant origin., of animal origin, of fungal origin or of bacterial origin and from laccases obtained by biotechnology.

5. Composition according to Claim 4, **characterized in that** the laccase is of plant origin and chosen from the laccases present in extracts of Anacardiacea plants, of Podocarpacea plants, of Rosmarinus off., of Solanum tuberosum, of Iris sp., of Coffea sp., of Daucus carrota, of Vinca minor, of Persea americana, of Catharanthus roseus, of Musa sp., of Malus pumila, of Gingko biloba, of Monotropa hypopithys (Indian pipe), of Aesculus sp., of Acer pseudoplatanus, of Prunus persica and of Pistacia palaestina.

6. Composition according to Claim 4, **characterized in that** the laccase is of microbial origin or obtained by biotechnology.

7. Composition according to Claim 6, **characterized in that** the laccase is chosen from laccases obtained from Polyporus versicolor, from Rhizoctonia praticola, from Rhus vernicifera, from Scytalidium, from Polyporus pinsitus, from Myceliophthora thermophila, from Rhizoctonia solani, from Pyricularia oryzae, from Trametes versicolor, from Fomes fomentarius, from Chaetomium thermophile, from Neurospora crassa, from Colorius versicolor, from Botrytis cinerea, from Rigidoporus lignosus, from Phellinus noxius, from Pleurotus ostreatus, from Aspergillus nidulans, from Podospora anserina, from Agaricus bisporus, from Ganoderma lucidum, from Glomerella cingulata, from Lactarius piperatus, from Russula delica, from Heterobasidion annosum, from Thelephora terrestris, from Cladosporium cladosporioides, from Cerrena unicolor, from Coriolus hirsutus, from Ceriporiopsis subvermispora, from Coprinus cinereus, from Panaeolus papilionaceus, from Panaeolus sphinctrinus, from Schizophyllum commune and from Dichomitius squalens, and from variants thereof.

8. Composition according to any one of the preceding claims, **characterized in that** the amount of laccase (s) is between 0.5 Lacu and 200 Lacu per 100 g of dye composition.

9. Composition according to any one of the preceding claims, **characterized in that** the coupler(s) is (are) chosen from meta-phenylenediamines, meta-aminophenols, meta-diphenols and heterocyclic couplers.

10. Composition according to Claim 9, **characterized in that** the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-N-(β-hydroxyethyl)amino-1-methoxybenzene, 1,3-diaminobenzene, 1,3-bis(2,4-diaminophenoxy)propane, sesamol, α-naphthol, 6-hydrbxyindole, 4-hydroxyindole, 4-hydroxy-N-methylindole, 6-hydroxyindoline, 2,6-dihydroxy-4-methylpyridine, 1-H-3-methylpyrazol-5-one, 1-phenyl-3-methylpyrazol-5-one, 2,6-dimethylpyrazolo[1,5-b]-1,2,4-triazole, 2,6-dimethyl[3,2-c]-1,2,4-triazole and 6-methylpyrazolo[1,5-a]benzimidazole, and the addition salts thereof with an acid.

11. Composition according to any one of the preceding claims, **characterized in that** the coupler(s) represent(s) from 0.0001% to 8% by weight relative to the total weight of the dye composition.

12. Composition according to Claim: 11, **characterized in that** the coupler(s) represent(s) from 0.005% to 5% by weight relative to the total weight of the dye composition.

13. Composition according to any one of the preceding claims, **characterized in that** it contains at least one additional oxidation base chosen from para-phenylenediamines, double bases, para-aminophenols, ortho-aminophenols and heterocyclic oxidation bases.

14. Composition according to Claim 13, **characterized in that** the additional oxidation base(s) represent(s) from 0.0005% to 12% by weight relative to the total weight of the dye Composition.

15. Composition according to Claim 14, **characterized in that** the additional oxidation base(s) represent(s) from 0.005% to 6% by weight relative to the total weight of the dye composition.

16. Composition according to any one of the preceding claims, **characterized in that** the addition salts with an acid are chosen from the hydrochlorides, hydrobromides, sulfates, tartrates, lactates and acetates.

17. Composition according to any one of the preceding claims, **characterized in that** the medium which is suitable for dyeing consists of water or of a mixture of water and at least one organic solvent.

18. Composition according to any one of the preceding claims, **characterized in that** it has a pH of between 4 and 11.

19. Process for dyeing keratin fibres, and in particular human keratin fibres such as the hair, **characterized in that** at least one ready-to-use dye composition as defined in any one of the preceding claims is applied to the said fibres, for a period which is sufficient to develop the desired coloration.

20. Process according to claim 19, **characterized in that** it includes a preliminary step which consists in separately storing, on the one hand, a composition (A) comprising, in a medium which is suitable for dyeing, 3-methyl-4-aminophenol and/or at least one addition salt thereof with an acid, as oxidation base, and, on the other hand, a composition (B) comprising, in a medium which is suitable for dyeing, at least one laccase enzyme, and then in mixing them together at the time of use, after which this mixture is applied to the keratin fibres.

21. Multi-compartment dyeing device or kit, **characterized in that** it includes a first compartment comprising composition (A) as defined in Claim 20 and a second compartment comprising composition (B) as defined in Claim 20.

## Patentansprüche

1. Gebrauchsfertige Zusammensetzung zum oxidativen Färben von Keratinfasern und insbesondere menschlichen Keratinfasern, wie Haaren, **dadurch gekennzeichnet, dass** sie in einem zum Färben geeigneten Medium enthält:
- das 3-Methyl-4-amino-phenol und/oder mindestens ein Additionssalz dieser Verbindung mit einer Säure als Oxidationsbase,
- mindestens ein Enzym vom Laccase-Typ, und
- mindestens einen Kuppler.

2. Zusammensetzung nach Anspruch 1, **dadurch gekennzeichnet, dass** das 3-Methyl-4-amino-phenol und/oder sein(e) Additionssalz(e) mit einer Säure 0,0005 bis 12 Gew.-% des Gesamtgewichts der Farbmittelzusammensetzung ausmachen.

3. Zusammensetzung nach Anspruch 2, **dadurch gekennzeichnet, dass** das 3-Methyl-4-amino-phenol und/oder sein(e) Additionssalz(e) mit einer Säure 0,005 bis 6 Gew.-% des Gesamtgewichts der Farbmittelzusammensetzung ausmachen.

4. Zusammensetzung nach einem der Ansprüche 1 bis 3, **dadurch gekennzeichnet, dass** die Laccase unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt ist.

5. Zusammensetzung nach Anspruch 4, **dadurch gekennzeichnet, dass** die Laccase pflanzlicher Herkunft ist und unter den Laccasen ausgewählt ist, die in Extrakten von Anacardiaceae, Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica und Pistacia palaestina enthalten sind.

6. Zusammensetzung nach Anspruch 4, **dadurch gekennzeichnet, dass** die Laccase mikrobieller Herkunft oder biotechnologisch hergestellt ist.

7. Zusammensetzung nach Anspruch 6, **dadurch gekennzeichnet, dass** die Laccase unter den Laccasen ausgewählt ist, die von Polyporus versicolor, Rhizoctonia praticola, Rhus vernicifera, Scytalidium, Polyporus pinsitus, Myceliophtora thermophila, Rhizoctonia solani, Pyricularia oryzae, Trametes versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

8. Zusammensetzung nach einem der vorhergehenden Ansprüche, **dadurch gekennzeichnet, dass** die Laccase(n) in einem Mengenanteil im Bereich von 0,5 bis 200 lacu pro 100 g Farbmittelzusammensetzung vorliegen.

9. Zusammensetzung nach einem der vorhergehenden Ansprüche, **dadurch gekennzeichnet, dass** der oder die Kuppler unter den m-Phenylendiaminen, m-Aminophenolen, m-Dihydroxybenzolen und heterocyclischen Kupplern ausgewählt sind.

10. Zusammensetzung nach Anspruch 9, **dadurch gekennzeichnet, dass** die Kuppler unter 2-Methyl-5-amino-phenol, 5-N-(β-Hydroxyethyl)-amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(β-hydroxyethyloxy)-benzol, 2-Amino-4-N-(β-hydroxyethylamino)-1-methoxy-benzol, 1,3-Diaminobenzol, 1,3-Bis(2,4-diaminophenoxy)-propan, Sesamol, α-Naphthol, 6-Hydroxyindol, 4-Hydroxyindol, 4-Hydroxy-N-methyl-indol, 6-Hydroxyindolin, 2,6-Dihydroxy-4-methyl-pyridin, 1-H-3-Methyl-pyrazol-5-on, 1-Phenyl-3-methyl-pyrazol-5-on, 2,6-Dimethylpyrazolo-[1,5-b]-1,2,4-triazol, 2,6-Dimethyl-[3,2-c]-1,2,4-triazol, 6-Methyl-pyrazolo-[1,5-a]-benzimidazol und den Additionssalzen dieser Verbindungen mit einer Säure ausgewählt sind.

11. Zusammensetzung nach einem der vorhergehenden Ansprüche, **dadurch gekennzeichnet, dass** der oder die Kuppler in einer Menge von 0,0001 bis 8 Gew.-% des Gesamtgewichts der Farbmittelzusammensetzung enthalten sind.

12. Zusammensetzung nach Anspruch 11, **dadurch gekennzeichnet, dass** der oder die Kuppler in einer Menge von 0,005 bis 5 Gew.-% des Gesamtgewichts der Farbmittelzusammensetzung enthalten sind.

13. Zusammensetzung nach einem der vorhergehenden Ansprüche, **dadurch gekennzeichnet, dass** sie mindestens eine zusätzliche Oxidationsbase enthält, die unter den p-Phenylendiaminen, Doppelbasen, p-Aminophenolen, o-Aminophenolen und den heterocyclischen Oxidationsbasen ausgewählt ist.

14. Zusammensetzung nach Anspruch 13, **dadurch gekennzeichnet, dass** die zusätzliche Oxidationsbase(n) in einer Menge von 0,0005 bis 12 Gew.-% des Gesamtgewichts der Zusammensetzung enthalten sind.

15. Zusammensetzung nach Anspruch 14, **dadurch gekennzeichnet, dass** die zusätzliche Oxidationsbase(n) in einer Menge von 0,005 bis 6 Gew.-% des Gesamtgewichts der Zusammensetzung enthalten sind.

16. Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

17. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

18. Zusammensetzung nach einem der vorhergehenden Ansprüche, **dadurch gekennzeichnet, dass** sie einen pH-Wert im Bereich von 4 bis 11 aufweist.

19. Verfahren zum Färben von Keratinfasern und insbesondere menschlichen Keratinfasern, wie dem Haar, **dadurch gekennzeichnet, dass** auf die Fasern mindestens eine gebrauchsfertige Farbmittelzusammensetzung nach einem der vorhergehenden Ansprüche während einer Zeitspanne, die zur Entwicklung der gewünschten Färbung ausreichend ist, aufgebracht wird.

20. Verfahren nach Anspruch 19, **dadurch gekennzeichnet, dass** es einen vorbereitenden Schritt umfasst, der darin besteht, einerseits eine Zusammensetzung (A), die in einem zum Färben geeigneten Medium das 3-Methyl-4-amino-phenol und/oder mindestens eines seiner Additionssalze mit einer Säure enthält, und andererseits eine Zusammensetzung (B) getrennt voneinander aufzubewahren, die in einem zum Färben geeigneten Medium mindestens ein Enzym vom Laccase-Typ enthält, und diese bei der Anwendung zu vermischen, bevor das Gemisch auf die Keratinfasern aufgebracht wird.

21. Vorrichtung mit mehreren Abteilungen oder "Kit" zum Färben, **dadurch gekennzeichnet, dass** sie eine erste Abteilung mit der in Anspruch 20 definierten Zusammensetzung (A) und eine zweite Abteilung mit der in Anspruch 20 definierten Zusammensetzung (B) enthält.
